# EUROPÄISCHE PATENTSCHRIFT

(11) **EP 2 005 924 B1**
(45) Veröffentlichungstag und Bekanntmachung des Hinweises auf die Patenterteilung: **29.08.2012**
(21) Anmeldenummer: 08017378.4
(22) Anmeldetag: 29.06.2004
(51) Int. Cl.: A61F 9/01, A61F 9/008, B23K 26/04, B23K 26/06

(54) **Vorrichtung zum Ausbilden von Schnittflächen in einem transparenten Material**
Device for forming cross-sections in a transparent material
Dispositif de développement d'interfaces dans un matériau transparent

(30) Priorität: 25.07.2003 DE 10334109
(43) Veröffentlichungstag der Anmeldung: 24.12.2008
(62) Teilanmeldung aus: 04763047.0
(73) Patentinhaber: Carl Zeiss Meditec AG, 07745 Jena (DE)
(72) Erfinder: Mühlhoff, Dirk, 07751 Jena (DE); Bischoff, Mark, Dr., 07749 Jena (DE); Gerlach, Mario, Dipl.-Ing., 16540 Hohen Neuendorf (DE); Lang, Carsten, 07607 Eisenberg (DE); Sticker, Markus, Dr., 07743 Jena (DE); Bergt, Michael, Dr., 99423 WEIMAR (DE)
(74) Vertreter: Geyer, Fehners & Partner

(56) Entgegenhaltungen:
- WO-A-03/082146
- DE-A- 10 162 166
- US-A- 6 099 522
- US-B1- 6 210 401

## Beschreibung

Die Erfindung bezieht sich auf eine Vorrichtung zum Ausbilden von Schnittflächen in einem transparenten Material, insbesondere in der Augenhomhaut, mlt einer Laserstrahlungsquelie, die Laserstrahlung in das Material fokussiert und dort optische Durchbrüche bewirkt, wobei eine Scaneinrichtung, die den Fokuspunkt verstellt, und eine Steuereinrichtung vorgesehen sind, die die Scaneinrichtung ansteuert, um die Schnittfläche durch eine flächengitferartige Anordnung aneinandergereihler optischer Durchbrüche im Material zu bilden, wobei die Steuereinrichtung den Fokuspunkt entllang einer Bahn verstellt und entlang der Bahn benachbarte optische Durchbrüche nicht unmittelbar hintereinander erzeugt.

Die US 62104011 beschreibt eine Vorrichtung zur Fehlsichtigkeitskorrektur mittels Hernhautablation. Gepulste Laserstrahlung wird in einem Muster auf die Hornhautoben fläche eingestrahlt, und das Muster wird mehrmals verschoben.

Gekrümmte Schnittflächen innerhalb eines transparenten Materials werden insbesondere bei laserchirurgischen Verfahren und dort inshesondere bei augenchirurgischen Eingriffen erzeugt. Dabei wird Behandlungs-laserstrahlung innerhalb des Gewebes d.h. unterhalb der Gewebeoberfläche derart fokussiert, daß optische Durchbrüche im Gewebe entstehen.

im Gewebe laufen dabei zeitlich hintereinander mehrere Prozesse ab, die durch die Lasersirahlung initiiert werden. Überschreitet die Leistungsdichie der Strahlung einen Schweilwert, komml es zu einem optischen Durchbruch, der im Material eine Plasmablase erzeugt. Diese Plasmablase wächst nach Entstehen des optischen Durchbruches durch sich ausdehnende Gase. Wird der optische Durchbruch nicht aufrechterhalten, so wird das In der Plasmablase erzeugte Gas vom umliegenden Material aufgenommen, und die Blase verschwindet wiader. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bet einer Plasmablase, die vorher verbundene Materialschichten trennt, ist üblicherweise von Photodisruption die Rede. Der Einfachneit halber werden alf solche Prozesse hier unter dem Begriff optischer. Durchbruch zusammengefaßt, d.h. dieser Begrift schließt nicht nur den eigentlichen optischen Durchbruch, sondern auch die daraus resultierenden Wirkungen im Material ein.

Für eine hohe Genauigkeit eines laserchirurgischen Verfahrens ist es unumgänglich, eine hohe Lokalisierung der Wirkung der Laserstrahlen zu gewährleisten und Kolateralschäden in benachbartem Gewebe möglichst zu vermeiden. Es ist deshalb im Stand der Technik üblich, die Laserstrahlung gepulst anzuwenden, so daß der zur Auslösung eines optischen Durchbruchs nötige Schwellwert für die Leistungsdichte nur in den einzelnen Pulsen überschritten wird. Die US 5.984.916 zeigt diesbezüglich deutlich, daß der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Material einzusetzen.

Der Einsatz von gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, daß die Brechungseigenschaften von Hornhaut und Linse keine optimale Fokussierung auf der Netzhaut bewirken.

Die erwähnte US 5.984.916 sowie die US 6.110.166 beschreiben Verfahren zur Schnitterzeugung mittels geeigneter Erzeugung optischer Durchbrüche, so daß im Endeffekt die Brechungseigenschaften der Hornhaut gezielt beeinflußt werden. Eine Vielzahl von optischen Durchbrüchen wird so aneinandergesetzt, daß innerhalb der Hornhaut des Auges ein linsenförmiges Teilvolumen isoliert wird. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, daß nach Entnahme die Form und damit die Brechungseigenschaften der Hornhaut so geändert sind, daß die erwünschte Fehlsichtigkeitskorrektur bewirkt ist. Die dabei geforderten Schnittflächen sind gekrümmt, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert.

Beim Aufbau eines Schnittes durch Aneinanderreihung optischer Durchbrüche im Material verläuft die Erzeugung eines optischen Durchbruches um ein Vielfaches schneller, als es dauert, bis ein davon erzeugtes Plasma wieder im Gewebe absorbiert wird. Aus der Veröffentlichung A. Heisterkamp et al., Der Ophthalmologe, 2001, 98:623-628, ist es bekannt, daß nach Erzeugen eines optischen Durchbruches in der Augenhornhaut am Fokuspunkt, an dem der optische Durchbruch erzeugt wurde, eine Plasmablase wächst, die nach einigen µs eine maximale Größe erreicht und anschließend wieder nahezu vollständig kollabiert. Es bleiben dann nur kleine Restblasen übrig. Die Veröffentlichung führt aus, daß ein Zusammenschließen anwachsender Plasmablasen die Schnittqualität mindert. Es wird deshalb dort ein gattungsgemäßes Verfahren vorgeschlagen, bei dem einzelne Plasmablasen nicht direkt nebeneinander erzeugt werden. Statt dessen wird in einer spiralförmigen Bahn zwischen aufeinanderfolgend erzeugten optischen Durchbrüchen eine Lücke gelassen, die in einem zweiten Durchlauf durch die Spirale mit optischen Durchbrüchen und daraus resultierenden Plasmablasen gefüllt wird. Damit soll ein Zusammenschluß benachbarter Plasmablasen verhindert und die Schnittqualität gefördert werden. Bei der von Heisterkamp et al. beschriebenen Spirale nimmt mit den Spiralwindungen der Abstand der erzeugten optischen Durchbrüche unvermeidlich zu.

Alternativ zu dem in der zitierten Veröffentlichung beschriebenen Vorgehen wäre es auch denkbar, den zeitlichen Abstand nacheinander erzeugter optischer Durchbrüche so groß zu gestalten, daß die Plasmablase eines optischen Durchbruches bereits kollabiert ist, bevor der nächste optische Durchbruch erzeugt wird. Dies würde jedoch die Schnittflächenerzeugung stark verlangsamen.

Eine schnelle Schnittflächenerzeugung ist nicht nur aus Komfort- oder Zeitersparniswünschen anzustreben; vor dem Hintergrund, daß bei ophthalmologischen Operationen unvermeidlicherweise Bewegungen des Auges auftreten, fördert eine schnelle Schnittflächenerzeugung auch die optische Qualität des erzielten Resultats bzw. senkt die Anforderungen an eventuelle Nachführungen von Augbewegungen.

Der Erfindung liegt deshalb die Aufgabe zugrunde, ein Verfahren und eine Vorrichtung der eingangs genannten Art so auszugestalten, daß für die Erzeugung einer qualitativ guten Schnittfläche eine möglichst geringe Zeit erforderlich ist.

Die Aufgabe wird mit einer Vorrichtung gemäß Anspruch 1 gelöst, bei der die flächengitterartige Anordnung der optischen Durchbrüche aus mindestens zwei Teilgittern aufgebaut ist und die Steuereinrichtung die Fokusverstellung so bewirkt, daß die Teilgitter hinsichtlich ihrer zugeordneten optischen Durchbrüche nacheinander abgearbeitet werden.

Die Erfindung erreicht durch Aufteilung der Schnittfläche in mehrere Teilgitter, daß zum einen bei der Aneinanderreihung der optischen Durchbrüche keine Gefahr besteht, direkt benachbarte optische Durchbrüche auch zeitlich direkt nacheinander zu erzeugen. Zum anderen ist eine vollständige bzw. gleichmäßige Füllung der Schnittfläche mit optischen Durchbrüchen erreicht.

Die durch Aneinanderreihung optischer Durchbrüche zu erzeugende Schnittfläche ist im allgemeinen gekrümmt. Auf der gekrümmten Fläche wird nun ein regelmäßiges Flächengitter derart definiert, daß eine gleichmäßige und vorzugsweise dichte Packung von Zonen, in denen optische Durchbrüche sich auswirken, erzielt wird. Dabei wird vorzugsweise darauf geachtet, daß der sphärische Abstand zwischen den Zentren zweier optischer Durchbrüche (auch als geodätische Linie bezeichnet) nur maximal 10 % größer ist, als der Abstand der Orte optischer Durchbrüche im Raum. Unter diesen Voraussetzungen kann ein kleines Gebiet der Schnittfläche in guter Näherung als ebener Flächenabschnitt angesehen werden. Unter "flächengitterartige Anordnung" wird deshalb die regelmäßige Anordnung der Orte, an denen optische Durchbrüche durch Fokussierung der Laserstrahlung initiiert werden, im dreidimensionalen Raum bezogen auf die Schnittfläche verstanden, wobei im Rahmen der oben erwähnten Näherung zumindest abschnittsweise von einem ebenen Flächenelement ausgegangen werden kann.

Durch geeignete Aufteilung der flächenhaften Anordnung der Plasmablasen in Teilgitter und sequentielle Abarbeitung der Teilgitter, d.h. es werden erst die Durchbrüche eines Teilgitters erzeugt, bevor die Durchbrüche des nächsten Teilgitters initiiert werden, ist erreicht, daß zwischen zwei zeitlich unmittelbar aufeinanderfolgend erzeugten Durchbrüchen immer ein räumlicher Abstand ist. Die Problematik des Zusammenwachsens von Plasmablasen direkt aufeinander erfolgter Durchbrüche ist vermieden. Dabei müssen einzelne Teilgitter nicht vollständig ausgeführt werden.

Mit steigender Geschwindigkeit, mit der optische Durchbrüche erzeugt werden, kann es auch zum Zusammenwachsen von Plasmablasen aus optischen Durchbrüchen kommen, die durch die Aneinanderreihung verschiedener Abschnitte der Bahnkurve benachbart sind. Die erfindungsgemäße Aufteilung der flächengitterartigen Anordnung in mindestens zwei Teilgitter erlaubt es diese Problematik zu vermeiden, da durch geeignete Wahl der Teilgitter dafür gesorgt werden kann, daß innerhalb eines Teilgitters keine optischen Durchbrüche in unmittelbarer Nachbarschaft erzeugt werden. Durch eine geeignete Wahl der Teilgitter kann weiter weitgehend für eine gleichmäßige Flächenfüllung gesorgt werden. Es ist in einer bevorzugten Ausführungsform der Erfindung vorgesehen, daß die Teilgitter so gewählt werden, daß in der flächengitterartigen Anordnung in mindestens einem Teilgitter für mindestens einen optischen Durchbruch alle benachbarten optischen Durchbrüche zu einem oder mehreren der anderen Teilgitter gehören. Zweckmäßigerweise wird man diesen Ansatz so ausführen, daß für alle Teilgitter die optischen Durchbrüche keinen unmittelbar benachbarten optischen Durchbruch, der zum selben Teilgitter gehört, haben. Durch diese Weiterbildung ist die Geschwindigkeit, mit der die Durchbrüche nacheinander erzeugt werden, hinsichtlich der Problematik des Zusammenwachsens von Plasmablasen nur noch durch den zeitlichen Abstand zweier Teilgitter beschränkt. Legt man die von Heisterkamp et al. publizierten Werte für das Anwachsen und Vergehen einer Plasmablase zugrunde, sollten zwischen dem den ersten Plasmablasen aufeinanderfolgenden Teilgittern mindestens etwa 2 bis 5 µs oder sogar einige Millisekunden bis Sekunden liegen.

Die Zahl an Teilgittern ist prinzipiell nicht beschränkt. Es hat sich jedoch gezeigt, daß bei zwei Teilgittern es mitunter nicht vollständig vermieden werden kann, daß zum selben Teilgitter gehörende Plasmablasen benachbart sind. Eine Aufteilung in drei Teilgitter ist deshalb vorteilhaft. Eine besonders vorteilhafte flächengitterartige Anordnung stellt das trigonale oder hexagonale Gitter dar (zur Anschaulichkeit werden hier die für das ebene Flächengitter üblichen Begriffe verwendet), bei dem nicht nur eine sehr hohe Flächenfüllung von über 90 % erreichbar ist, sondern bei dem auch auf einfache Weise sichergestellt ist, daß ein zu einem Teilgitter gehörender optischer Durchbruch nur unmittelbare Nachbarn aus den anderen beiden Teilgittern hat.

Die verschiedenen Teilgitter lassen sich zweckmäßigerweise durch ein Grundteilgitter erzeugen, das entsprechend der Anzahl an Teilgittern geeignet verschoben wird, um die flächengitterartige Anordnung zu erreichen. Dieser Ansatz hat weiter den Vorteil, daß die Steuereinrichtung die Fokusverstellung gemäß einem dem Grundteilgitter zugeordneten festen Schema vornehmen kann, beispielsweise in Form einer bestimmten Abtastbahn bzw. eines bestimmten Rastermoduses, und für die einzelnen Teilgitter lediglich eine relativ einfache Koordinatentransformation, beispielsweise in Form einer Verschiebung berücksichtigen muß.

Für die erfindungsgemäße Vorrichtung kommt jede geeignete Steuereinheit in Frage, die das erläuterte Verfahren ausführt, beispielsweise ein geeignet programmierter Mikroprozessor oder Computer, der die Baugruppen der Vorrichtung geeignet ansteuert.

Die Erfindung wird nachfolgend unter Bezugnahme auf die Zeichnung beispielhalber noch näher erläutert. In der Zeichnung zeigt:
- Figur 1: eine perspektivische Darstellung eines Patienten während einer laserchirurgischen Behandlung mit einem laserchirurgischen Instrument,
- Figur 2: die Fokussierung eines Strahlenbündels auf das Auge des Patienten beim Instrument der Figur 1,
- Figur 3: eine schematische Darstellung zur Erläuterung einer während der laserchirurgischen Behandlung mit dem Instrument der Figur 1 erzeugten Schnittfläche,
- Figur 4: eine Ablenkvorrichtung des laserchirurgischen Instruments der Figur 1,
- Figur 5: drei Teilfiguren 5a, 5b und 5c zum Aufbau der Schnittfläche der Figur 3 aus mehreren Teilgittern.

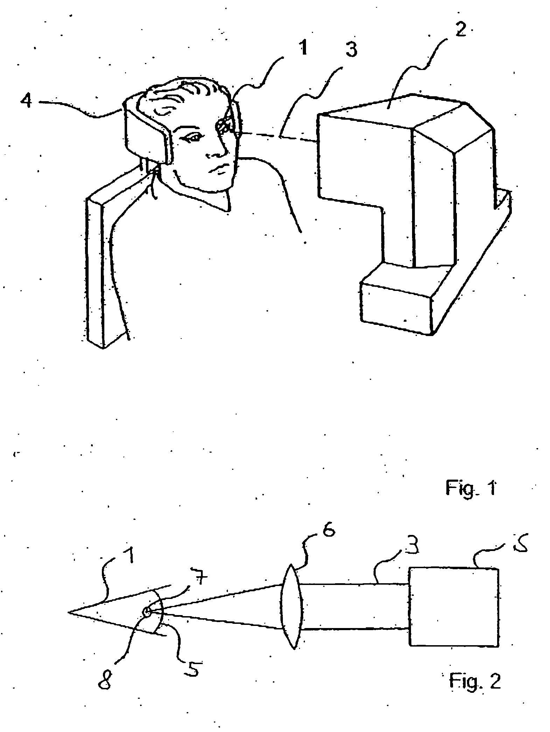

In Figur 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einen Kopfhalter 4 fixiert ist. Das laserchirurgische Instrument 2 ist in der Lage, einen gepulsten Laserstrahl 3 zu erzeugen, so daß das in US 6.110.166 beschriebene Verfahren ausgeführt werden kann. Die Baugruppen des Instrumentes 2 werden von einer im Ausführungsbeispiel integrierten Steuereinheit gesteuert.

Das laserchirurgische Instrument 2 weist dazu, wie in Figur 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, daß aus der Hornhaut 5 Material so entfernt wird, daß sich die Brechungseigenschaften der Hornhaut um ein gewünschtes Maß ändern. Das Material wird dabei dem Stroma der Hornhaut entnommen, das unterhalb von Epithel und Bowmanscher Membran oberhalb der Decemetschen Membran und des Endothels liegt.

Die Materialentfernung erfolgt, indem durch Fokussierung des hochenergetischen gepulsten Laserstrahls 3 mittels eines verstellbaren Teleskopes 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher eine Plasmablase 8 initiiert. Dadurch umfaßt die Gewebeschichttrennung ein größeres Gebiet, als der Fokus 7 der Laserstrahlung 3. Durch geeignete Ablenkung des Laserstrahls 3 werden nun während der Behandlung viele Plasmablasen 8 generiert. Diese Plasmablasen 8 bilden dann eine Schnittfläche 9, die ein Teilvolumen T des Stromas, nämlich das zu entfernende Material der Hornhaut 5 umschreiben.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man den Schnitt durch weitere Erzeugung von Plasmablasen 8 bis an die Oberfläche der Hornhaut 5, kann ein durch die Schnittfläche 9 isoliertes Material der Hornhaut 5 seitlich herausgezogen und somit entfernt werden.

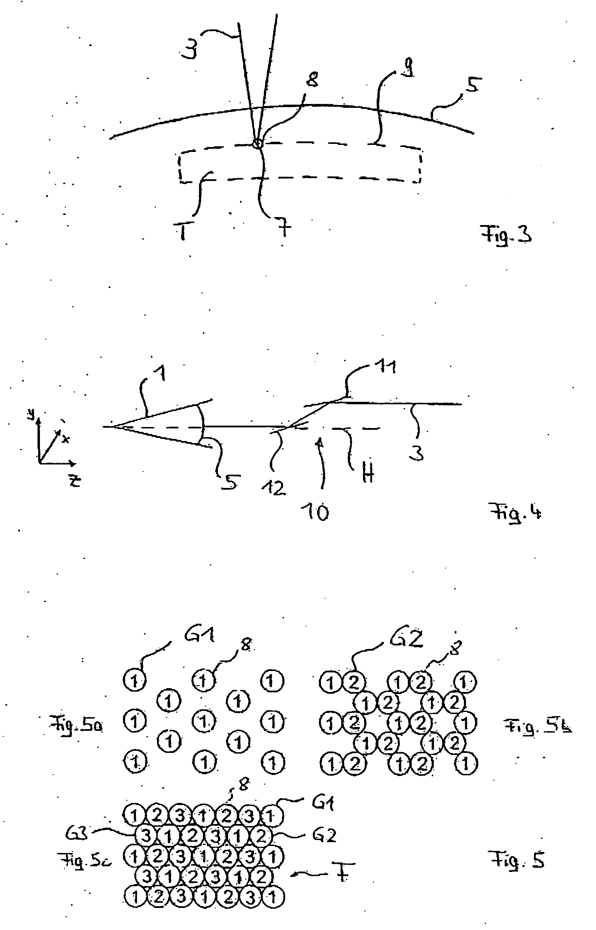

Die Erzeugung der Schnittfläche 9 mittels des laserchirurgischen Instrumentes 2 ist in Figur 3 schematisch dargestellt. Durch Aneinanderreihung der Plasmablasen 8 in Folge stetiger Verschiebung des Fokus 7 des gepulsten fokussierten Laserstrahls 3 wird die Schnittfläche 9 gebildet.

Die Fokusverschiebung erfolgt dabei zum einen in einer Ausführungsform mittels der in Figur 4 schematisch dargestellten Ablenkeinheit 10, die den auf einer Haupteinfallsachse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der Hauptstrahlachse H mit der Ablenkachse ist dann der jeweilige Ablenkpunkt. Zur Fokusverschiebung wird zum anderen das Teleskop 6 geeignet verstellt. Dadurch kann der Fokus 7 in dem in Figur 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der y-Richtung zu verstellen erlaubt. Das Teleskop 6 wirkt dagegen auf die z-Koordinate des Fokus 7.

Aufgrund der Corneakrümmung, die zwischen 7 und 10 mm beträgt, ist das Teilvolumen T auch entsprechend gekrümmt. Die Corneakrümmung wirkt sich somit in Form einer Bildfeldkrümmung aus. Diese wird durch geeignete Ansteuerung der Ablenkeinheit 10 und des Teleskopes 6 berücksichtigt.

Ist eine wie in Figur 3 gezeigte Schnittfläche in die gleiche Richtung wie die Hornhautoberfläche gewölbt, so ist dies mit einer Optik, deren Bildfeldkrümmung ähnlich der Krümmung der Hornhaut ist, zu erreichen, ohne daß die Führung des Fokus 7 dies berücksichtigen muß.

Die gekrümmte Schnittfläche 9 wird durch Aneinanderreihung von Plasmablasen 8 durch geeignete Verstellung des Fokus 7 sowie Ansteuerung der Strahlquelle S erzeugt. Dabei kann beispielshalber ein Abrastern der Schnittfläche 9 erfolgen. Die Steuereinrichtung des Instrumentes 2 steuert die Ablenkeinrichtung 10 und die Scanoptik 6 jedoch so an, daß innerhalb eines gewissen Zeitfensters keine Durchbrüche in unmittelbarer Nachbarschaft entstehen. Die Anordnung der Plasmablasen 8, die die Schnittfläche 9 bilden, kann als (gekrümmte) flächengitterartige Anordnung F aufgefaßt werden. Zur Veranschaulichung ist in Figur 5 eine ebene Darstellung für die flächengitterartige Anordnung F gewählt, realiter sind die einzelnen Plasmablasen 8 natürlich auf einer räumlich gekrümmten Fläche angeordnet, um das Teilvolumen T zu isolieren. Die derart auf der gekrümmten Schnittfläche 9 definierte flächengitterartige Anordnung F wird nun nicht durch unmittelbar sequentielles Abarbeiten der darin liegenden Plasmablasen 8 erzeugt, statt dessen unterteilt die Steuereinheit des Instrumentes 2 die flächengitterartige Anordnung F in drei Teilgitter G1, G2 und G3, die in den Figuren 5a bis 5c gezeigt sind. Die Teilgitter G1 und G2 sowie G3 sind dabei aus einem gemeinsamen Grundgitter gewonnen, das jeweils um den Abstand zwischen zwei Plasmablasen 8 entlang einer Gitterachse verschoben ist.

Die Steuereinrichtung fährt nun die einzelnen Punkte der flächengitterartigen Anordnung F rasterartig so ab, daß zuerst die Punkte des Teilgitters G1 abgearbeitet werden. Ist an jedem Punkt des Teilgitters G1 eine Plasmablase 8 erzeugt, nimmt die Steuereinheit eine Koordinatenverschiebung bezüglich des Rastermusters des Teilgitters G1 vor und erzeugt optische Durchbrüche gemäß dem Teilgitter G2. Die optischen Durchbrüche 8 des Teilgitters G2 sind zwar optischen Durchbrüchen des Teilgitters G1 jeweils unmittelbar benachbart, haben jedoch keine unmittelbaren Nachbarn innerhalb des Teilgitters G2. Bei geeigneter Wahl der Teilgitter G1 und G2 ist die Schnittfläche 9 dann fertig. Bei der in der Figur gezeigten Aufteilung ist dagegen noch ein drittes Teilgitter G3 vorgesehen.

Eine nochmalige Koordinatentransformation der Steuereinheit des Instrumentes 2 sorgt dafür, daß in einem dritten Durchlauf an den Punkten des Teilgitters G3 optische Durchbrüche erzeugt werden, die jeweils die noch verbleibenden Lücken zwischen den Plasmablasen 8 der Teilgitter G1 und G2 füllen. Im Ergebnis ist die flächengitterartige Anordnung F vollständig mit Plasmablasen 8 gefüllt, so daß die Schnittfläche 9 abgeschlossen ist.

Das Zugrundelegen eines Teilgitters bei der Abarbeitung der Punkte, an denen Plasmablasen 8 für die Schnittfläche 9 initiiert werden müssen, hat den Vorteil, daß die Steuereinheit des Instrumentes 2 mit einem festen Ablenkschema arbeiten kann, das zum Abarbeiten der Teilgitter G1, G2 und G3 lediglich einer festen Koordinantentransformation unterworfen werden muß. Gleichzeitig ist sichergestellt, daß kein Punkt der flächengitterartigen Anordnung F ohne Plasmablase 8 bleibt.

## Patentansprüche

1. Vorrichtung zum Ausbilden von Schnittflächen (9) in einem transparenten Material, insbesondere in der Augenhornhaut (5), mit einer Laserstrahlungsquelle (S), die Laserstrahlung (3) in das Innere des Materials fokussiert und dort optische Durchbrüche (8) bewirkt, wobei eine Scaneinrichtung (6, 10), die den Fokuspunkt (7) verstellt, und eine Steuereinrichtung (2) vorgesehen sind, die so ausgebildet ist, daß sie die Scaneinrichtung (6, 10) ansteuert, um die Schnittfläche (9) durch eine flächengitterartige Anordnung (F) aneinandergereihter optischer Durchbrüche (8) im Material (5) zu bilden, wobei die Steuereinrichtung (2) so ausgebildet ist, daß sie den Fokuspunkt (7) entlang einer Bahn verstellt und entlang der Bahn benachbarte optische Durchbrüche (8) nicht unmittelbar hintereinander erzeugt, wobei die flächengitterartige Anordnung (F) der optischen Durchbrüche (8) aus mindestens zwei Teilgittern (G1, G2, G3) aufgebaut ist, die Steuereinrichtung (2) so ausgebildet ist, daß sie die Fokusverstellung so bewirkt, daß die Teilgitter (G1, G2, G3) hinsichtlich ihrer zugeordneten optischen Durchbrüche (8) nacheinander abgearbeitet werden und wobei zwischen aufeinanderfolgend erzeugten optischen Durchbrüchen (8) jedes Teilgitter (G1, G2, G3) ein räumlicher Abstand ist, so daß die durch die aufeinanderfolgenden optischen Durchbrüche (8) im Material erzeugten Plasmablasen nicht zusammenwachsen.

2. Vorrichtung nach Anspruch 1, **dadurch gekennzeichnet, daß** die Steuereinrichtung (2) die drei Teilgitter (G1, G2, G3) so wählt, daß in mindestens einem Teilgitter (G1, G2, G3) für mindestens einen optischen Durchbruch (8) alle benachbarten optischen Durchbrüche (8) zu anderen Teilgittern (G1, G2, G3) gehören.

3. Vorrichtung nach Anspruch 2, **dadurch gekennzeichnet, daß** für alle Teilgitter (G1, G2, G3) die optischen Durchbrüche (8) keinen unmittelbar benachbarten optischen Durchbruch (8), der zum selben Teilgitter (G1, G2, G3) gehört, haben.

4. Vorrichtung nach einem der Ansprüche 1, 2 oder 3, **dadurch gekennzeichnet, daß** die Steuereinrichtung (2) die Schnittfläche (9) durch eine flächengitterartige Anordnung in Form eines trigonalen Gitters erzeugt und die drei Teilgitter (G1, G2, G3) aus einem Grundgitter in drei verschiedenen Verschiebungen entlang einer Achse des Grundteilgitters erzeugt.

5. Verfahren oder Vorrichtung nach einem der obigen Ansprüche, **dadurch gekennzeichnet, daß** mindestens ein Teilgitter (G1, G2, G3) nicht vollständig mit optischen Durchbrüchen (8) abgearbeitet wird.

## Claims

1. A device for producing cuts (9) in a transparent material, in particular in the cornea of the eye (5), said device comprising a source of laser radiation (S), which focuses laser radiation (3) into the material and causes optical breakthroughs (8) therein, wherein a scanning unit (6, 10), which shifts the focal point (7), and a control unit (2) which controls the scanning unit (6, 10), and is adapted to control the scanning unit (6, 10) for forming the cut (9) by a surface lattice-type array (F) of arranged optical breakthroughs (8) in the material (5), said control unit (2) being adapted to shift the focal point (7) along a path and to generating optical breakthroughs (8) adjacent along said path not immediately one after the other, wherein the surface lattice-type array (F) of the optical breakthroughs (8) is made up of at least two partial lattices (G1, G2, G3) and the control unit (2) is adapted to effect focus shifting such that the partial lattices (G1, G2, G3) are processed after each other, with respect to their associated optical breakthroughs (8), and wherein there is a gap between sequentially generated optical breakthroughs (8) of each partial lattice (G1, G2, G3), the gap being such that plasma bubbles generated in the material by sequential optical breakthroughs (8) do not merge.

2. Device according to claim 1, **characterized in that** the control unit (2) selects three partial lattices (G1, G2, G3) such that, in at least one partial lattice (G1, G2, G3) for at least one optical breakthrough (8) all adjacent optical breakthroughs (8) belong to other partial lattices (G1, G2, G3).

3. Device according to claim 2, **characterized in that**, for all partial lattices (G1, G2, G3), the optical breakthroughs (8) do not have an immediately adjacent optical breakthrough (8) belonging to the same partial lattice (G1, G2, G3).

4. Device according to any one of claims 1, 2 or 3, **characterized in that** the control unit (2) generates the cut (9) by a surface lattice-type array in the form of a trigonal lattice and generates the three partial lattices (G1, G2, G3) from a lattice template by three different displacements of the template along an axis of said partial lattice template.

5. Method or device according to any one of the above claims, **characterized in that** at least one partial lattice (G1, G2, G3) is not processed completely with optical breakthroughs (8).

## Revendications

1. Dispositif de réalisation de surfaces de coupe (9) dans une matière transparente, en particulier dans la cornée (5), comprenant une source de rayonnement laser (S) qui focalise le rayonnement laser (3) vers l'intérieur de la matière et y crée des passages optiques (8), dans lequel un dispositif de balayage (6, 10) qui ajuste le point focal (7) et un dispositif de commande (2) réalisé de telle sorte qu'il pilote le dispositif de balayage (6, 10) sont prévus pour former la surface de coupe (9) dans la matière (5) par un agencement (F) de type réseau plan, composé de passages optiques (8) juxtaposés, dans lequel le dispositif de commande (2) est réalisé de telle sorte qu'il ajuste le point focal (7) le long d'une trajectoire et génère le long de la trajectoire des passages optiques (8) voisins de manière non directement consécutive, dans lequel l'agencement (F) de type réseau plan des passages optiques (8) se compose d'au moins deux réseaux partiels (G1, G2, G3), le dispositif de commande (2) est réalisé pour qu'il provoque l'ajustement du point focal de telle sorte que les réseaux partiels (G1, G2, G3) sont traités successivement concernant leurs passages optiques (8) attribués, et dans lequel il existe une distance physique entre des passages optiques (8) de chaque réseau partiel (G1, G2, G3) générés successivement de telle sorte que des bulles de plasma générées dans la matière par les passages optiques (8) consécutifs ne s'agglutinent pas.

2. Dispositif selon la revendication 1, **caractérisé en ce que** le dispositif de commande (2) choisit les trois réseaux partiels (G1, G2, G3) de telle sorte que dans au moins un réseau partiel (G1, G2, G3), pour au moins un passage optique (8), tous les passages optiques (8) voisins appartiennent à des réseaux partiels (G1, G2, G3) différents.

3. Dispositif selon la revendication 2, **caractérisé en ce que** pour tous les réseaux partiels (G1, G2, G3), les passages optiques (8) ne présentent aucun passage optique (8) directement voisin et appartenant au même réseau partiel (G1, G2, G3).

4. Dispositif selon l'une quelconque des revendications 1, 2 ou 3, **caractérisé en ce que** le dispositif de commande (2) génère la surface de coupe (9) par un agencement de type réseau plan sous la forme d'un réseau trigonal et génère les trois réseaux partiels (G1, G2, G3) à partir d'un réseau de base en trois translations différentes le long d'un axe du réseau partiel de base.

5. Procédé ou dispositif selon l'une quelconque des revendications précédentes, **caractérisé en ce qu'**au moins un réseau partiel (G1, G2, G3) n'est pas traité complètement avec des passages optiques (8).
